(19) Europäisches Patentamt
European Patent Office
Office européen des brevets

(11) EP 1 902 713 A1

(12) **EUROPEAN PATENT APPLICATION**
published in accordance with Art. 153(4) EPC

(43) Date of publication:
**26.03.2008 Bulletin 2008/13**

(21) Application number: **06780873.3**

(22) Date of filing: **07.07.2006**

(51) Int Cl.:
***A61K 31/427*** *(2006.01)* ***A61K 9/20*** *(2006.01)*
***A61K 47/32*** *(2006.01)* ***A61K 47/38*** *(2006.01)*
***A61P 3/10*** *(2006.01)* ***C07D 417/12*** *(2006.01)*

(86) International application number:
**PCT/JP2006/313548**

(87) International publication number:
**WO 2007/007656 (18.01.2007 Gazette 2007/03)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.07.2005 JP 2005199661**
**23.01.2006 JP 2006013679**

(71) Applicant: **Daiichi Sankyo Company, Limited**
**Tokyo 103-8426 (JP)**

(72) Inventors:
- **KAWASAKI, Takao**
**Shinagawa-ku**
**Tokyo 140-8710 (JP)**
- **SUZUKI, Nobuyuki**
**Shinagawa-ku**
**Tokyo 140-8710 (JP)**
- **YADA, Shuichi**
**Shinagawa-ku**
**Tokyo 140-8710 (JP)**

(74) Representative: **Gibson, Christian John Robert**
**Marks & Clerk**
**90 Long Acre**
**London WC2E 9RA (GB)**

(54) **PHARMACEUTICAL COMPOSITION CONTAINING THIAZOLIDINEDIONE COMPOUND**

(57) [OBJECT]

To provide a pharmaceutical composition containing a thiazolidinedione compound and having superior solubility.

[MEANS FOR SOLUTION]

A pharmaceutical composition incorporating cellulose, a cellulose derivative, a polyvinyl alcohol derivative, a polyvinyl alcohol derivative mixture or a mixture thereof with 5-{4-[(6-methoxy-1-methyl-1H-benzimidazol-2-yl) methoxy]benzyl}thiazolidine-2,4-dione, or a pharmacologically acceptable salt thereof.

[Fig. 1]

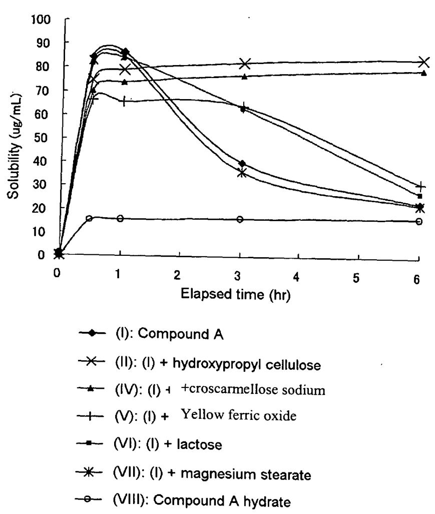


EP 1 902 713 A1

## Description

[TECHNICAL FIELD]

The present invention relates to a pharmaceutical composition containing a thiazolidinedione compound and having superior solubility.

[BACKGROUND ART]

Insulin sensitizers are used as effective medications against diabetes and the like since they have the action of improving insulin resistance and lowering blood glucose levels. Examples of insulin sensitizers currently sold commercially include pioglitazone (Takeda Pharmaceutical Co., Ltd.) and rosiglitazone (GlaxoSmithKline K.K.).

In addition, although an insulin sensitizer in the form of a pharmaceutical composition containing 5-{4-[(6-methoxy-1-methyl-1H-benzimidazol-2-yl)methoxy]benzyl}thiazolidine-2,4-dione, or a pharmacologically acceptable salt thereof, is disclosed in Patent Document 1 and Patent Document 2, there is no description whatsoever regarding effect s attributable to excipients.

[Patent Document 1] Pamphlet of International Publication No. WO 00/71540
[Patent Document 2] Pamphlet of International Publication No. PCT/JP2006/301058

[DISCLOSURE OF THE INVENTION]

[Problems to be Solved by the Invention]

Since insulin sensitizers are required to have high absorbability into the body as a consequence of the mechanism of action thereof, they are required to have a high level of solubility that remains stable over time. However, highly pure 5-{4-[(6-methoxy-1-methyl-1H-benzimidazol-2-yl)methoxy]benzyl}thiazolidine-2,4-dione hydrochloride (hereinafter referred to as Compound A) has been determined to undergo hydrate formation over time in a water-containing solvent, and due to the extremely low solubility of that hydrate, there has been concern that Compound A demonstrates a decrease in absorbability of active ingredient into the body due to this hydrate formation occurring in the gastrointestinal tract following administration.

Therefore, as a result of conducting extensive studies on the stability of a pharmaceutical composition containing highly pure 5-{4-[(6-methoxy-1-methyl-1H-benzimidazol-2-yl)methoxy]benzyl}thiazolidine-2,4-dione, or a pharmacologically acceptable salt thereof, the inventors of the present invention found that a pharmaceutical composition incorporating as an excipient cellulose, a cellulose derivative, a polyvinyl alcohol derivative, a polyvinyl alcohol derivative mixture or a mixture thereof with 5-{4-[(6-methoxy-1-methyl-1H-benzimidazol-2-yl)methoxy]benzyl}thiazolidine-2,4-dione, or a pharmacologically acceptable salt thereof, is useful as a pharmaceutical formulation for warm-blooded animals (particularly humans) as a result of having superior solubility and stability of solubility over time, thereby leading to completion of the present invention.

[Means for Solving the Problems]

The present invention is:

(1) a pharmaceutical composition containing highly pure 5-{4-[(6-methoxy-1-methyl-1H-benzimidazol-2-yl)methoxy]benzyl}thiazolidine-2,4-dione, or a pharmacologically acceptable salt thereof, and one type or two or more types of excipients selected from pharmacologically acceptable cellulose, cellulose derivatives, polyvinyl alcohol derivatives and a polyvinyl alcohol derivative mixture;
(2) the pharmaceutical composition according to (1) above, wherein a decrease in solubility of the highly pure 5-{4-[(6-methoxy-1-methyl-1H-benzimidazol-2-yl)methoxy]benzyl}thiazolidine-2,4-dione, or pharmacologically acceptable salt thereof, is prevented;
(3) the pharmaceutical composition according to (1) above, wherein hydrate formation of the highly pure 5-{4-[(6-methoxy-1-methyl-1H-benzimidazol-2-yl)methoxy]benzyl}thiazolidine-2,4-dione or pharmacologically acceptable salt thereof, is prevented;
(4) the pharmaceutical composition according to (1) above, wherein superior in vivo absorbability of the 5-{4-[(6-methoxy-1-methyl-1H-benzimidazol-2-yl)methoxy]benzyl}thiazolidine-2,4-dione, or pharmacologically acceptable salt thereof, is maintained by preventing a decrease in the solubility of the highly pure 5-{4-[(6-methoxy-1-methyl-1H-benzimidazol-2-yl)methoxy]benzyl}thiazolidine-2,4-dione, or pharmacologically acceptable salt thereof;

(5) the pharmaceutical composition according to (1) above, wherein hydrate formation of the highly pure 5-{4-[(6-methoxy-1-methyl-1H-benzimidazol-2-yl)methoxy]benzyl}thiazolidine-2,4-dione, or pharmacologically acceptable salt thereof, is prevented in a water-containing solution;
(6) the pharmaceutical composition according to (1) above, wherein the highly pure 5-{4-[(6-methoxy-1-methyl-1H-benzimidazol-2-yl)methoxy]benzyl}thiazolidine-2,4-dione, or pharmacologically acceptable salt thereof, has superior solubility and stability of solubility over time in a water-containing solution;
(7) the pharmaceutical composition according to (1) above, wherein a decrease in solubility of the highly pure 5-{4-[(6-methoxy-1-methyl-1H-benzimidazol-2-yl)methoxy]benzyl}thiazolidine-2,4-dione, or pharmacologically acceptable salt thereof, is prevented in a water-containing solution;
(8) the pharmaceutical composition according to (1) above, wherein a decrease in solubility of the highly pure 5-{4-[(6-methoxy-1-methyl-1H-benzimidazol-2-yl)methoxy]benzyl}thiazolidine-2,4-dione, or pharmacologically acceptable salt thereof, is prevented in the gastrointestinal tract of a warm-blooded animal;
(9) the pharmaceutical composition according to any one of (1) to (8) above that is a formulation for oral administration;
(10) the pharmaceutical composition according to (9) above, wherein the formulation for oral administration is a tablet;
(11) the pharmaceutical composition according to any one of (1) to (10) above, wherein the 5-{4-[(6-methoxy-1-methyl-1H-benzimidazol-2-yl)methoxy]benzyl}thiazolidine-2,4-dione, or pharmacologically acceptable salt thereof, is a purified composition in which the content of the 5-{4-[(6-methoxy-1-methyl-1H-benzimidazol-2-yl)methoxy]benzyl}thiazolidine-2,4-dione, or pharmacologically acceptable salt thereof, excluding water, is 98% by weight or more;
(12) the pharmaceutical composition according to any one of (1) to (10) above, wherein the 5-{4-[(6-methoxy-1-methyl-1H-benzimidazol-2-yl)methoxy]benzyl}thiazolidine-2,4-dione, or pharmacologically acceptable salt thereof, is a purified composition in which the content of the 5-{4-[(6-methoxy-1-methyl-1H-benzimidazol-2-yl)methoxy]benzyl}thiazolidine-2,4-dione, or pharmacologically acceptable salt thereof, excluding water, is 99% by weight or more.
(13) the pharmaceutical composition according to any one of (1) to (12) above, wherein the excipient is a pharmacologically acceptable cellulose or cellulose derivative;
(14) the pharmaceutical composition according to (13) above, wherein the pharmacologically acceptable cellulose or cellulose derivative is one or more types selected from methyl cellulose, hydroxymethyl cellulose, hydroxyethyl cellulose, hydroxyethyl methyl cellulose, hydroxypropyl cellulose, hydroxypropyl methyl cellulose 2208, hydroxypropyl methyl cellulose 2906, hydroxypropyl methyl cellulose 2910 and croscarmellose sodium;
(15) the pharmaceutical composition according to any one of (1) to (13) above, wherein the excipient is a pharmacologically acceptable polyvinyl alcohol derivative;
(16) the pharmaceutical composition according to (15) above, wherein the pharmacologically acceptable polyvinyl alcohol derivative is partially hydrolyzed polyvinyl alcohol;
(17) the pharmaceutical composition according to any one of (1) to (16) above, wherein the 5-{4-[(6-methoxy-1-methyl-1H-benzimidazol-2-yl)methoxy]benzyl}thiazolidine-2,4-dione, or pharmacologically acceptable salt thereof, is 5-{4-[(6-methoxy-1-methyl-1H-benzimidazol-2-yl)methoxy]benzyl}thiazolidine-2,4-dione hydrochloride;
(18) the pharmaceutical composition according to any one of (1) to (17) above that is produced by a dry granulation process;
(19) the pharmaceutical composition according to any one of (1) to (17) above that is produced by a wet granulation process;
(20) the pharmaceutical composition according to any one of (1) to (19) that is for the treatment of diabetes;
(21) use of 5-{4-[(6-methoxy-1-methyl-1H-benzimidazol-2-yl) methoxy]benzyl}thiazolidine-2,4-dione, or a pharmacologically acceptable salt thereof, and one type or two or more types of excipients selected from cellulose, cellulose derivatives, polyvinyl alcohol derivatives and polyvinyl alcohol derivative mixtures for manufacturing the pharmaceutical composition according to any one of (1) to (20) above;
(22) use of 5-{4-[(6-methoxy-1-methyl-1H-benzimidazol-2-yl)methoxy]benzyl}thiazolidine-2,4-dione, or a pharmacologically acceptable salt thereof, and a pharmacologically acceptable cellulose or cellulose derivative for manufacturing the pharmaceutical composition according to any one of (1) to (21) above;
(23) use of a purified composition, in which the content of 5-{4-[(6-methoxy-1-methyl-1H-benzimidazol-2-yl)methoxy] benzyl}thiazolidine-2,4-dione, or a pharmacologically acceptable salt thereof, excluding water, is 98% by weight or more, for manufacturing the pharmaceutical composition according to any one of (1) to (21) above;
(24) use of the pharmaceutical composition according to any one of (1) to (21) above for treating diabetes;
(25) use of a pharmaceutical composition containing highly pure 5-{4-[(6-methoxy-1-methyl-1H-benzimidazol-2-yl) methoxy]benzyl}thiazolidine-2,4-dione, or a pharmacologically acceptable salt thereof, and a pharmacologically acceptable cellulose or cellulose derivative for treating diabetes;
(26) a method for administering 5-{4-[(6-methoxy-1-methyl-1H-benzimidazol-2-yl)methoxy] benzyl}thiazolidine-2,4-dione, or a pharmacologically acceptable salt thereof, comprising: co-administering (i) a purified composition in which the content of 5- {4-[(6-methoxy-1-methyl-1H-benzimidazol-2-yl)methoxy]benzyl} thiazolidine-2,4-dione, or a pharmacologically acceptable salt thereof, excluding water, is 98% by weight or more, and (ii) one type or two or

more types selected from a polyvinyl alcohol derivative and a polyvinyl alcohol derivative mixture to a patient requiring the 5-{4-[(6-methoxy-1-methyl-1H-benzimidazol-2-yl)methoxy]benzyl}thiazolidine-2,4-dione, or a pharmacologically acceptable salt thereof;

(27) the method according to (26) above for improving in vivo absorbability by maintaining the solubility of highly pure 5-{4-[(6-methoxy-1-methyl-1H-benzimidazol-2-yl)methoxy]benzyl}thiazolidine-2,4-dione, or a pharmacologically acceptable salt thereof;

(28) a method for treating a human to treat or prevent diabetes, the method comprising: treating the human with a pharmaceutical composition containing (i) highly pure 5-{4-[(6-methoxy-1-methyl-1H-benzimidazol-2-yl)methoxy]benzyl}thiazolidine-2,4-dione, or a pharmacologically acceptable salt thereof, and (ii) one type or two or more types of excipients selected from cellulose, cellulose derivatives, polyvinyl alcohol derivatives and polyvinyl alcohol derivative mixtures; and,

(29) the method according to (28) above, comprising treating with a pharmaceutical composition containing (i) highly pure 5-{4-[(6-methoxy-1-methyl-1H-benzimidazol-2-yl)methoxy]benzyl}thiazolidine-2,4-dione, or a pharmacologically acceptable salt thereof, and (ii) a pharmacologically acceptable cellulose or derivative thereof.

The active ingredient of the pharmaceutical composition of the present invention in the form of 5-{4-[(6-methoxy-1-methyl-1H-benzimidazol-2-yl)methoxy]benzyl}thiazolidine-2,4-dione, or a pharmacologically acceptable salt thereof, is a compound represented by the following structure, or a pharmacologically acceptable salt thereof, and is described in the aforementioned Patent Documents 1 and 2.

[CHEMICAL 1]

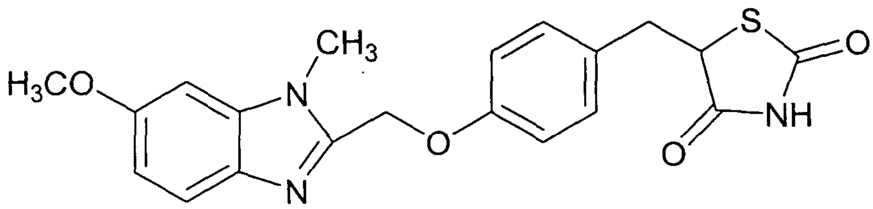

It is preferably 5-{4-[(6-methoxy-1-methyl-1H-benzimidazol-2-yl)methoxy]benzyl}thiazolidine-2,4-dione hydrochloride.

Examples of cellulose and cellulose derivatives serving as excipients of the pharmaceutical composition of the present invention include microcrystalline cellulose (Avicel: Asahi Kasei Corp., etc.), microcrystalline cellulose•carmellose sodium (Avicel RC: Asahi Kasei Corp., etc.), methyl cellulose (Metolose SM: Shin-Etsu Chemical Co., Ltd., etc.), ethyl cellulose (Ethocel: Dow Chemical Co., etc.), hydroxypropyl cellulose (Nisso HPC: Nippon Soda Co., Ltd., etc.), low-substituted hydroxypropyl cellulose (L-HPC: Shin-Etsu Chemical Co., Ltd., etc.), hydroxypropyl methyl cellulose 2208 (Metolose 90SH: Shin-Etsu Chemical Co., Ltd., etc.), hydroxypropyl methyl cellulose 2906 (Metolose 65SH: Shin-Etsu Chemical Co., Ltd., etc.), hydroxypropyl methyl cellulose 2910 (Metolose 60SH: Shin-Etsu Chemical Co., Ltd., etc.), hydroxypropyl cellulose phthalate 200731 (HPMCP: Shin-Etsu Chemical Co., Ltd., etc.), hydroxypropyl cellulose phthalate 220824 (HPMCP: Shin-Etsu Chemical Co., Ltd., etc.), hydroxypropyl methyl cellulose acetate succinate (Shin-Etsu AQOAT: Shin-Etsu Chemical Co., Ltd., etc.), carmellose (NS-300: Gotoku Chemical Co., Ltd., etc.), carmellose calcium (ECG-505: Gotoku Chemical Co., Ltd., etc.), carmellose sodium (Cellogen: Daiichi Kogyo Seiyaku Co., Ltd., etc.), croscarmellose sodium (Ac-Di-Sol: Asahi Kasei Corp., etc.), carboxymethyl ethyl cellulose (CMEC: Freund Corp., etc.), cellulose acetate phthalate (CAP: Wako Pure Chemical Industries, Ltd., etc.), hydroxyethyl cellulose (NATROSOL: Aqualon Corp., etc.) or mixtures thereof, and more preferably include methyl cellulose, hydroxypropyl cellulose, hydroxypropyl methyl cellulose 2208, hydroxypropyl methyl cellulose 2906, hydroxypropyl methyl cellulose 2910, carmellose sodium, croscarmellose sodium or mixtures thereof.

Examples of "polyvinyl alcohol derivatives" and "polyvinyl alcohol derivative mixtures" include fully hydrolyzed polyvinyl alcohol, partially hydrolyzed polyvinyl alcohol and mixtures containing the same, while more preferable examples include partially hydrolyzed polyvinyl alcohol (GOHSENOL GL-05, Nippon Synthetic Chemical Industry Co., Ltd., etc.).

A highly pure substance in the present invention refers to a substance having a content of the main component thereof of 97% by weight or more.

In the present invention, "content" refers to a quantitative value (wt%) as measured by quantitative analysis (preferably the quantitative analysis method described in Reference Example 2). Quantitative values can be determined, for example, from a surface area ratio followed by measurement of an object substance by HPLC.

The amount of cellulose, cellulose derivatives, polyvinyl alcohol derivatives, polyvinyl alcohol derivative mixtures or mixtures thereof used as an excipient in the present invention is normally 0.1 to 30 parts by weight and preferably

0.5 to 25 parts by weight.

Although the pharmaceutical composition of the present invention can be used in the form of an orally administerable formulation such as tablets, capsules, pills, granules or grains, it is preferably used in the form of a tablet.

The pharmaceutical composition of the present invention may suitably contain a pharmaceutically acceptable excipient. Examples of such excipients include diluents (for example, sugar derivatives such as lactose, sucrose, glucose, mannitol or sorbitol; starch derivatives such as cornstarch, potato starch, partially pregelatinized starch, dextrin, carboxymethyl starch or sodium carboxymethyl starch; pregelatinized starch; cellulose derivatives such as microcrystalline cellulose, methyl cellulose, hydroxypropyl cellulose, low-substituted hydroxypropyl cellulose, hydroxypropyl methyl cellulose, carmellose, carmellose calcium, croscarmellose or croscarmellose sodium; gum Arabic; dextran; pullulan; silicate derivatives such as light silicic anhydride, calcium silicate, silicate hydrate, synthetic aluminum silicate or magnesium aluminometasilicate; phosphate derivatives such as dicalcium phosphate; chloride derivatives such as sodium chloride; carbonate derivatives such as calcium carbonate; sulfate derivatives such as calcium sulfate; or mixtures thereof, preferably sugar derivatives, cellulose derivatives or mixtures thereof, and more preferably lactose, mannitol, microcrystalline cellulose or mixtures thereof), binders (for example, compounds listed as examples of the aforementioned diluents; gelatin; polyvinylpyrrolidone; Macrogol; or, mixtures thereof, preferably cellulose derivatives or mixtures thereof, and more preferably hydroxypropyl cellulose), disintegrants (for example, compounds listed as examples of the aforementioned diluents; crospovidone; or, mixtures thereof, preferably cellulose derivatives or mixtures thereof, and more preferably low-substituted hydroxypropyl cellulose, croscarmellose sodium or mixtures thereof), lubricants (for example, stearic acid; stearic acid metal salts such as calcium stearate and magnesium stearate; benzoic acid metal salts such as sodium benzoate; waxes such as beegum and spermaceti; boric acid; glycol; carboxylic acids such as fumaric acid and adipic acid; sulfuric acid metal salts such as sodium sulfate; leucine; lauryl sulfuric acid metal salts such as sodium lauryl sulfate and magnesium lauryl sulfate; silicate derivatives listed as examples of the aforementioned diluents; starch derivatives listed as examples of the aforementioned diluents; hydrogenated vegetable oils; carnauba wax; sucrose fatty acid esters; or, mixtures thereof, preferably stearic acid metal salts, silicate derivatives or mixtures thereof, and more preferably calcium stearate, magnesium stearate, silicic anhydride or mixtures thereof), stabilizers (for example, benzoic acid; benzoic acid metal salts such as sodium benzoate; paraoxybenzoic acid esters such as methyl paraben and propyl paraben; alcohols such as chlorobutanol, benzyl alcohol and phenylethyl alcohol; benzalkonium chloride; phenols such as phenol and cresol; thimerosal; acetic anhydride; sorbic acid: or, mixtures thereof, preferably benzoic acid metal salts, paraoxybenzoic acid esters or mixtures thereof, and more preferably sodium benzoate, methyl paraben, propyl paraben or mixtures thereof), fluidizing agents (for example, silicate derivatives listed as examples of the aforementioned diluents; talc; or, mixtures thereof, and preferably light silicic anhydride, talc or mixtures thereof), surfactants (for example, polysorbates such as polysorbate 80; polyoxyethylene hydrogenated castor oils such as polyoxyethylene hydrogenated castor oil 60; sorbitan fatty acid esters; sucrose fatty acid esters; polyoxyethylene polyoxypropylene glycols; polyoxyethylene fatty acid ethers; polyoxyl stearates; or, mixtures thereof, and preferably polysorbate 80, polyoxyethylene hydrogenated castor oil 60 or mixtures thereof), colorants (for example, yellow ferric oxide, ferric oxide and black iron oxide), antioxidants, correctives (for example, commonly used sweeteners, sour flavorings and fragrances), or diluents, and although varying according to the tablet, capsule or other administration form, the types and amounts of excipients used are selected according to known technologies in the field of pharmacology.

For example, in the case of tablets, the content of binder in the entire pharmaceutical composition is normally 1 to 10 parts by weight (and preferably 2 to 5 parts by weight), the content of disintegrant is normally 1 to 40 parts by weight (and preferably 5 to 30 parts by weight), the content of lubricant is normally 0.1 to 10 parts by weight (and preferably 0.5 to 3 parts by weight), and the content of fluidizing agent is normally 0.1 to 10 parts by weight (and preferably 0.5 to 5 parts by weight).

The pharmaceutical composition of the present invention is easily produced according to a known manufacturing method (such as a dry manufacturing method, kneading using water or wet granulation) using pharmacologically acceptable excipients.

"Wet granulation " in the present invention refers to a process for obtaining a formulation by granulating a powder using water or a mixture of water and alcohol as a granulation binder, and is described in publications such as The Theory and Practice of Industrial Pharmacy (Third Edition) (Leon Lachman, et al.: LEA & FEBIGER 1986) and Pharmaceutical Dosage Forms: Tablets Volume 1 (Second Edition) (Herbert A. Lieberman, et al.: MARCEL DEKKER INC. 1989). Here, granulation refers to a procedure for manufacturing granules having a nearly uniform shape and size from a raw material in the form of a powder, mass, solution or melting liquid and the like, and includes granulation for manufacturing a finished product such as granules, powders or fine granules, and granulation for manufacturing a production intermediate product such as tablets or capsules.

As an example of the production thereof, an active ingredient, stabilizer, diluent, binder, disintegrant, and as necessary, other types of assistants and the like are added followed by mixing with a high shear granulator, and an aqueous solution of a binder are then added to the resulting mixture followed by kneading to obtain granules. The resulting granules are then dried with a fluid bed dryer, and the dried granules are forcibly passed through a screen

using a screening mill followed by the addition of lubricant, disintegration agent, and as necessary, other assistants and the like, mixing with a V-blender, and forming the resulting mixture into tablets or packing into capsules to produce tablets or capsules, respectively.

A dry manufacturing method in the present invention consists of direct compression and dry granulation. "Direct compression" refers to obtaining a formulation by directly compressing and molding a raw material powder. "Dry granulation" refers to compressing and molding a raw material powder into the shape of a slag or sheet, crushing with a suitable method, and obtaining a formulation by using the granules produced. These processes are described in publications such as The Theory and Practice of Industrial Pharmacy (Third Edition) (Leon Lachman, et al.: LEA & FEBIGER 1986) and Pharmaceutical Dosage Forms: Tablets Volume 1 (Second Edition) (Herbert A. Lieberman, et al.: MARCEL DEKKER INC. 1989).

The resulting tablets can be sugar-coated or film-coated (preferably film-coated) as necessary. For example, the resulting tablets can be coated with a film by spraying the tablets with a coating liquid composed of hydroxypropyl methyl cellulose, polyvinyl alcohol, talc, titanium oxide, lactose, triacetine or polyethylene glycol, yellow ferric oxide or ferric oxide and water in a pan coating machine.

In addition, a mixture obtained by mixing with the aforementioned screening mill, adding an aqueous solution of binder and kneading can be formed into granules using an extrusion granulator followed by drying with an air-through tray dryer and forcibly passing the dried granules through a screen using a screening mill to produce a granules.

The pharmaceutical composition of the present invention can be administered to warm-blooded animals (particularly humans), and although able to be varied according to various conditions such as patient symptoms, age and body weight, the dosage of the active ingredient in the form of 5-{4-[(6-methoxy-1-methyl-1H-benzimidazol-2-yl)methoxy]benzyl}thiazolidine-2,4-dione, or a pharmacologically acceptable salt thereof, is 0.001 to 1 mg/kg (preferably 0.005 to 0.05 mg/kg) per administration to an adult, administered one to three times per day according to symptoms in the case of oral administration.

[Effects of the Invention]

According to the present invention, a pharmaceutical composition is provided having superior stability of solubility over time and superior drug absorbability by preventing the formation of a hydrate of 5-{4-[(6-methoxy-1-methyl-1H-benzimidazol-2-yl)methoxy]benzyl}thiazolidine-2,4-dione, or a pharmacologically acceptable salt thereof.

[BEST MODE FOR CARRYING OUT THE INVENTION]

The present invention is described in more detail by way of examples, formulation examples and reference examples thereof, but the present invention is not limited thereto.

The 5-{4-[(6-methoxy-1-methyl-1H-benzimidazol-2-yl)methoxy]benzyl}thiazolidine-2,4-dione hydrochloride used in the present invention (hereinafter referred to as Compound A) can be produced according to the method of Reference Example 1. A monohydrate of Compound A can be produced according to the method of Reference Example 3. The content of Compound A can be confirmed according to the method of Reference Example 2.

The formulation excipients used in the present invention are commercially available products. OPADRY II 85F48993 (manufactured by Colorcon, Inc., hereinafter referred to as OPADRY II) is a premixed product containing polyvinyl alcohol, masking agent, plasticizer and the like, OPAGLOS 2 (manufactured by Colorcon, Inc.) is a premixed product containing carmellose sodium, masking agent, anti-adhesion agent and the like, and OPADRY WHITE YS-1-18202A (manufactured by Colorcon, Inc., hereinafter referred to as OPADRY WHITE) is a premixed product containing hydroxypropyl methyl cellulose, masking agent and plasticizer.

[Examples]

(Test Example 1)

Dissolution Rate Test 1 of 5-{4-[(6-methoxy-1-methyl-1H-benzimidazol-2-yl)methoxy]benzyl}thiazolidine-2,4-dione hydrochloride (Compound A)

About 40 mg of Compound A or a monohydrate of Compound A and a formulation excipient [one excipient is selected from the following excipients: 377.6 mg of lactose (Borculo Domo Ingredients), 78.0 mg of croscarmellose sodium (Ac-Di-Sol, FMC International), 15.6 mg of hydroxypropyl cellulose (HPC-L, Nippon Soda Co., Ltd.), 5.2 mg of magnesium stearate (NOF Corp.) and 0.112 mg of yellow ferric oxide (Kishi Kasei Co., Ltd.)] were added to 200 ml of Japanese Pharmacopoeia (JP) first fluid (7.0 ml of hydrochloric acid and water added to 2.0 g of sodium chloride followed by dissolving and bringing to a volume of 100 ml, normally used as a test liquid that imitates disintegration and elution

of chemicals in the stomach) followed by stirring with a stirrer at 37°C in a 300 ml conical beaker. 10 ml-samples were collected after 30 minutes, 1 hour, 3 hours and 6 hours followed by filtration using Acrodisk LC13 (PVDF, Gelman Science, Inc.). After discarding the first 3 ml, the next 7 ml were removed into a test tube. 5 ml of the sample in the test tube was taken with a whole pipette and transferred to a test tube containing 2 ml of methanol followed by mixing.

Quantification was carried out using HPLC, and solubility was determined from a calibration curve generated according to the method described below.

The calibration curve was generated by preparing methanol standard solutions of Compound A at concentrations of 400, 100 and 20 μg/ml, followed by the addition of 5 ml of JP first fluid to 2 ml of each standard solution, mixing and quantifying by HPLC.

HPLC Conditions:

Analytical column: L-column ODS (4.6 mm ID x 15 cm, Chemicals Evaluation and Research Institute, Japan)
Mobile phase: 0.01 mol/l mixture of acetate buffer (pH 5.0) and acetonitrile (13:7)
Flow rate: 1.0 ml/min
Column temperature: 40°C
Detector: Ultraviolet absorption spectrophotometer (measuring wavelength: 290 nm)

The results of measuring the solubility of Compound A are shown in Fig. 1.

As shown in Fig. 1, in the case of Compound A only (I), solubility was observed to decrease over time. The cause of this decrease in solubility is thought to be Compound A changing to a hydrate over time in JP first fluid, and since the solubility of this monohydrate is extremely low (VIII), the solubility of Compound A ended up decreasing over time.

Therefore, the effects of various types of excipients were examined for the purpose of preventing a decrease in solubility of Compound A, and those results are shown in Table 1. In Table 1, test mixture groups containing excipients containing cellulose and/or a derivative thereof consist of groups (II) and (IV) (hereinafter collectively referred to as the "Cellulose-Containing Group"), while test mixture groups not containing excipients containing cellulose and/or a derivative thereof consist of groups (V), (VI) and (VII) (hereinafter collectively referred to as the "Non-Cellulose-Containing Groups").

There were no remarkable differences observed in solubility between the Cellulose-Containing Group and Non-Cellulose- Containing Group up to 1 hour after the start of the solubility test. However, the solubilities of Compound A and the Non-Cellulose-Containing Group subsequently decreased rapidly. On the other hand, the solubility of the Cellulose-Containing Group continued to maintain high solubility.

(Test Example 2)

Dissolution Rate Test 2 of 5-{4-[(6-methoxy-1-methyl-1H-benzimidazol-2-yl)methoxy]benzyl}thiazolidine-2,4-dione hydrochloride (Compound A)

About 40 mg of Compound A or a monohydrate of Compound A and a formulation excipient [one excipient is selected from the following excipients: 23.9 mg of OPADRY II, 17.2 mg of polyvinyl alcohol (GOHSENOL GL-05, Nippon Synthetic Chemical Industry Co., Ltd.), and 23.9 mg of OPAGLOS 2] were added to 200 ml of Japanese Pharmacopoeia (JP) first fluid (7.0 ml of hydrochloric acid and water added to 2.0 g of sodium chloride followed by dissolving and bringing to a volume of 100 ml, normally used as a test liquid that imitates disintegration and elution of chemicals in the stomach) followed by stirring with a stirrer at 37°C in a 300 ml conical beaker. 10 ml-samples were collected after 30 minutes, 1 hour, 3 hours and 6 hours followed by filtration using Acrodisk LC13 (PVDF, Gelman Science, Inc.). After discarding the first 3 ml, the next 7 ml were removed into a test tube. 5 ml of the sample in the test tube was taken with a whole pipette and transferred to a test tube containing 2 ml of methanol followed by mixing.

Quantification was carried out using HPLC, and solubility was determined from a calibration curve generated according to the method described below.

The calibration curve was generated by preparing methanol standard solutions of Compound A at concentrations of 400, 100 and 20 μg/ml, followed by the addition of 5 ml of JP first fluid to 2 ml of each standard solution, mixing and quantifying by HPLC.

HPLC Conditions:

Analytical column: L-column ODS (4.6 mm ID x 15 cm, Chemicals Evaluation and Research Institute, Japan)
Mobile phase: 0.01 mol/l mixture of acetate buffer (pH 5.0) and acetonitrile (13:7)
Flow rate: 1.0 ml/min
Column temperature: 40°C
Detector: Ultraviolet absorption spectrophotometer (measuring wavelength: 290 nm)

The results of measuring the solubility of Compound A are shown in Fig. 2.

There were no remarkable differences observed in solubility between the Compound A Group and the excipient Group to 1 hour after the start of the solubility test. However, the solubility of the Compound A Group subsequently decreased rapidly. On the other hand, the solubility of the excipient Group containing cellulose derivative or polyvinyl alcohol continued to maintain high solubility.

On the basis of these results, high solubility was determined to be able to be stably maintained by adding an excipient consisting of a cellulose derivative, polyvinyl alcohol derivative, polyvinyl alcohol derivative mixture or mixture thereof to Compound A. The reasons for obtaining these results are thought to be that the aforementioned excipients inhibit Compound A from changing to a hydrate, and that the solubility of hydrated Compound A is enhanced.

Thus, a pharmaceutical composition containing Compound A and an excipient in the form of cellulose, cellulose derivative, polyvinyl alcohol derivative, polyvinyl alcohol derivative mixture or mixture thereof is able to enhance absorption of Compound A into the body as a result of maintaining high solubility, thereby resulting in an extremely superior pharmaceutical composition.

(Formulation Example 1)

Manufacturing of 5-{4-[(6-methoxy-1-methyl-1H-benzimidazol-2-yl)methoxy]benzyl}thiazolidine-2,4-dione hydrochloride (Compound A) Tablets by Wet Granulation

Tablets containing Compound A and cellulose or a derivative thereof were obtained according to the process described below using the types and amounts of components shown in Table 1. Furthermore, the dosage of the active ingredient, content of each excipient and type of each excipient are not limited to those shown in Table 1.

A diluent (lactose) and a disintegrant (croscarmellose sodium (Ac-Di-Sol)) were added to Compound A followed by mixing with a high sheer granulator, adding an aqueous solution of binder (hydroxypropyl cellulose) to the resulting mixture and kneading to obtain granules. The resulting granules were dried using a fluid bed dryer, the dried granules were forcibly passed through a screen using a screening mill and a lubricant (magnesium stearate) was added followed by mixing with a V-blender. The resulting mixture was molded using a punch having a diameter of 7 mm followed by spraying with an aqueous coating solution (OPADRY WHITE) in which a pigment (yellow ferric oxide) was dispersed using a coating machine to obtain the desired tablets.

(Table 1)

| Component | Amount per tablet (mg) |
|---|---|
| Compound A | 10.9 |
| Lactose | 94.4 |
| Ac-Di-Sol | 19.5 |
| Hydroxypropyl cellulose | 3.9 |
| Magnesium stearate | 1.3 |
| OPADRY WHITE | 5.972 |
| Yellow ferric oxide | 0.028 |
| Total | 136.0 |

(Formulation Example 2)

Manufacturing of 5-{4-[(6-methoxy-1-methyl-1H-benzimidazol-2-yl)methoxy]benzyl}thiazolidine-2,4-dione hydrochloride (Compound A) Tablets by Dry Granulation

Tablets containing Compound A and cellulose or a derivative thereof were obtained according to the process described below using the types and amounts of components shown in Table 2. Furthermore, the dosage of the active ingredient, content of each excipient and type of each excipient are not limited to those shown in Table 2.

A diluent (lactose (Dilactose S)), a disintegrant (croscarmellose sodium (Ac-Di-Sol)) and a binder (hydroxypropyl cellulose) were added to Compound A followed by mixing with a V-blender, passing the resulting mixture through a 30 mesh sieve and additionally mixing with a V-blender to obtain a mixture. A lubricant (magnesium stearate) was then added to the resulting mixture and mixed with a V-blender. The resulting mixture was molded using a punch having a diameter of 7 mm followed by spraying with an aqueous coating solution (OPADRY WHITE) in which a pigment (yellow ferric oxide) was dispersed using a coating machine to obtain the desired tablets.

(Table 2)

| Component | Amount per tablet (mg) |
|---|---|
| Compound A | 10.9 |
| Lactose | 94.4 |
| Ac-Di-Sol | 19.5 |
| Hydroxypropyl cellulose | 3.9 |
| Magnesium stearate | 1.3 |
| OPADRY WHITE | 5.972 |
| Yellow feric oxide | 0.028 |
| Total | 136.0 |

(Reference Example 1)

Manufacturing of 5-{4-[(6-methoxy-1-methyl-1H-benzimidazol-2-yl)methoxy]benzyl}thiazolidine-2,4-dione hydrochloride

(1-1) Acetonitrile (140.9 kg) was added to 4-[(2,4-dioxothiazolidin-5-yl)methyl]phenoxyacetic acid (18.0 kg, 64.0 mol) produced according to the process described in Japanese Patent Application (Kokai) No. 2001-72671, and after cooling to an internal temperature of 8°C, thionyl chloride (8.3 kg, 69.8 mol) was added. Dimethylformamide (14.4 L) was further added followed by stirring for 3.5 hours at a temperature of 8 to 15°C. An acetonitrile (84.6 kg) solution of tert-butyl N-(2-amino-5-methoxyphenyl)-N-methylcarbamate (15.7 kg, 62.2 mol) and triethylamine (8.4 kg, 83.0 mol) held at a temperature of 0 to 10°C was added dropwise thereto over 1 hour while cooling so as to maintain at a temperature of 0 to 5°C followed by further stirring for 2 hours at the same temperature. Next, water (144 L) was added over 22 minutes followed by stirring for 30 minutes while holding at an internal temperature of 0 to 6°C and allowing to stand undisturbed for 12 hours. After filtering out the resulting crystals, the crystals were washed with a 2:1 aqueous solution (54 L) to obtain wet crystals of tert-butyl N- {2-{4-(2,4-dioxothiazolidin-5-yl) methyl]phenoxyacetylamino}-5-methoxyphenyl}-N-methylcarbamate.

(1-2) A suspension of the wet crystals of tert-butyl N-{2-{4-(2,4-dioxothiazolidin-5-yl)methyl]phenoxyacetylamino}-5-methoxyphenyl}-N-methylcarbamate obtained in (1-1) in methanol (450 L) was refluxed for 25 minutes while stirring. The suspension was cooled to 0 to 5°C followed by stirring for 1 hour at the same temperature and filtering out the precipitated crystals. The resulting crystals were washed with methanol (54 L) to obtain wet purified crystals of wet crystals of tert-butyl N-{2-{4-[(2,4-dioxothiazolidin-5-yl)methyl]phenoxyacetylamino}-5-methoxyphenyl} -N-methylcarbamate.

(1-3) The wet purified crystals of wet crystals of tert-butyl N-{2-{4-[(2,4-dioxothiazolidin-5-yl)methyl]phenoxyacetylamino} -5-methoxyphenyl}-N-methylcarbamate obtained in (1-2) were suspended in methanol (1080 L) and refluxed to obtain a solution. This solution was then cooled to 50°C and filtered over 45 minutes. The residue was washed with methanol (37 L) at 50°C. The filtrate and washings were combined followed by pouring in 38% hydrochloric acid (20.9 kg) at 48°C and then water (11.2 L) and stirring for 6 hours at the same temperature. After cooling the reaction solution to 0 to 5°C, it was stirred for 30 minutes at the same temperature and allowed to stand undisturbed for 12 hours. The resulting crystals were filtered out and washed with methanol (91 L) followed by drying at 50°C under reduced pressure to obtain 5-{4-[(6-methoxy-1-methyl-1H-benzimidazol-2-yl)methoxy]benzyl}thiazolidine-2,4-dione hydrochloride (19.5 kg, 44.9 mol) (cumulative yield of Reference Example 1: 70%).

(Reference Example 2)

Quantitative Analysis of 5-{4-[(6-methoxy-1-methyl-1H-benzimidazol-2-yl)methoxy]benzyl}thiazolidine-2,4-dione hydrochloride (Compound A)

The following describes quantitative analysis of Compound A. The difference between (2-1) and (2-2) is a correction for the water content contained in the purified composition of Compound A.

(2-1) Quantitative Analysis of 5-{4-[(6-methoxy-1-methyl-1H-benzimidazol-2-yl)methoxy]benzyl}thiazolidine-2,4-dione hydrochloride (Compound A) - Method 1

Sample dissolving solution: Acetonitrile-0.5% aqueous phosphoric acid (7:13)
Internal standard solution: About 1 ml of methyl salicylate dissolved in the sample dissolving solution brought to a final volume of 200 ml.

The standard solution was prepared in the manner described below.

About 0.04 g of Compound A standard was accurately weighed into a 200 ml volumetric flask, and after adding about 160 ml of sample dissolving solution and dissolving, 10 ml of internal standard solution and sample dissolving solution were added to bring to a final volume of 200 ml. 10 ml of this solution was accurately transferred to a 25 ml volumetric flask and brought to a final volume of 25 ml by addition of sample dissolving solution.

The measuring sample solution was prepared in the manner described below.

About 0.04 g of sample was weighed into a 200 ml volumetric flask, and after adding about 160 ml of sample dissolving solution and dissolving, 10 ml of internal standard solution and sample dissolving solution were added to bring to a final volume of 200 ml. 10 ml of this solution was accurately transferred to a 25 ml volumetric flask followed by bringing to a final volume of 25 ml by addition of sample dissolving solution.

HPLC conditions were as indicated below.
Detection wavelength: 290 nm
Column: L-column ODS, 4.6 x 150 mm (Chemicals Evaluation and Research Institute, Japan)
Mobile phase: 0.01 M mixture of sodium acetate buffer (pH = 5) and acetonitrile (13:7)
Flow rate: 1 ml/min (adjusted so that the retention time of the internal standard was about 17 minutes)
Column temperature: 40°C

The area of each peak was determined from the chromatogram, and the content of Compound A was calculated according to the equation indicated below.

$$\text{Content of Compound A (\%)} = W1 \times F1 \times (QT/QS) \times [1/\{W2 \times (100 - F2)/100\}] \times 100$$

W1: Weighed amount of Compound A standard (g)
W2: Weighed amount of sample (g)
F1: Purity coefficient of Compound A standard
F2: Moisture in sample as determined with Karl Fischer moisture meter
QT: Ratio of peak areas between sample and internal standard
QS: Ratio of peak areas between Compound A standard and internal standard

As a result of analyzing according to the procedure described above, the quantitative analysis value of Compound A used in Test Example 1 was 99.1 % by weight. The quantitative analysis value of the crystals of Reference Example 1 was 99.4% by weight.

(2-2) Quantitative Analysis of 5-{4-[(6-methoxy-1-methyl-1H-benzimidazol-2-yl)methoxy]benzyl}thiazolidine-2,4-dione hydrochloride (Compound A) - Method 2

Sample dissolving solution: Acetonitrile-0.5% aqueous phosphoric acid (7:13)
Internal standard solution: About 1 g of methyl salicylate dissolved in the sample dissolving solution (acetonitrile- water mixture 7:13) brought to a final volume of 200 ml.

The standard solution was prepared in the manner described below.

About 0.02 g of Compound A standard was accurately weighed into a 50 ml volumetric flask, and after adding about 40 ml of sample dissolving solution and dissolving, 5 ml of internal standard solution and sample dissolving solution were added to bring to a final volume of 50 ml. 10 ml of this solution was accurately transferred to a 50 ml volumetric flask and brought to a final volume of 50 ml by addition of sample dissolving solution.

The measuring sample solution was prepared in the manner described below.

About 0.02 g of sample was weighed into a 50 ml volumetric flask, and after adding about 40 ml of sample dissolving solution and dissolving, 5 ml of internal standard solution and sample dissolving solution were added to bring to a final volume of 50 ml. 10 ml of this solution was accurately transferred to a 50 ml volumetric flask followed by bringing to a final volume of 50 ml by addition of sample dissolving solution.

HPLC conditions were as indicated below.

Detection wavelength: 290 nm
Column: L-column ODS, 4.6 x 150 mm (Chemicals Evaluation and Research Institute, Japan)
Mobile phase: 0.01 M mixture of sodium acetate buffer (pH = 5) and acetonitrile (13:7)
Flow rate: 1 ml/min (adjusted so that the retention time of the internal standard was about 10 minutes)
Column temperature: 40°C

The area of each peak was determined from the chromatogram, and the content of Compound A was calculated according to the equation indicated below.

$$\text{Content of Compound A (\%)} = W1 \times F1 \times [(100 - F2)/100] \times (QT/QS) \times (1/W2) \times 100$$

W1: Weighed amount of Compound A standard (g)
W2: Weighed amount of sample (g)
F1: Purity coefficient of Compound A standard
F2: Moisture content of Compound A standard (%)
QT: Ratio of peak areas between sample and internal standard
QS: Ratio of peak areas between Compound A standard and internal standard

As a result of analyzing according to the procedure described above, the quantitative analysis value of Compound A used in Test Example 2 was 98.2% by weight.

(Reference Example 3)

Manufacturing of 5-{4-[(6-methoxy-1-methyl-1H-benzimidazol-2-yl)methoxy]benzyl}thiazolidine-2,4-dione hydrochloride (Compound A) monohydrate

An acetone suspension (700 ml) of tert-butyl N-[2-[4-(2,4-dioxothiazolidin-5-yl)methyl]phenoxyacetylamino]-5-methoxyphenyl]-N-methylcarbamate (28.0 g, 54.31 mmol) produced according to the process described in Japanese Patent Application (Kokai) No. 2001-72671 was heated to 55°C followed by the addition of water (234 ml) to the resulting solution. 36% hydrochloric acid (46.2 ml) was added to this solution at the same temperature and the reaction mixture was stirred for 5.5 hours at 55 to 58°C. After cooling the reaction suspension to 25°C, the precipitated crystals were filtered out and washed with a mixed solvent of acetone (58.8 ml) and water (25.2 ml). The resulting crystals were dried at 55°C under reduced pressure to obtain the desired compound (22.87 g, 50.61 mmol). Yield: 93%

Differential thermal analysis: About 77°C and about 99°C (heat absorption corresponding to dehydration), about 203°C (heat absorption), about 243°C (heat absorption), about 281°C (heat absorption)
$^1$H-NMR (500MHz, DMSO-$d_6$) $\delta$ (ppm): 3.15 (1H, dd, J = 9.2 Hz, J = 14.1 Hz), 3.39 (1H, dd, J = 4.5 Hz, J = 14.1 Hz), 3.94 (3H, s), 4.03 (3H, s), 4.95 (1H, dd, J = 4.5 Hz, J = 9.2 Hz), 5.67 (2H, s), 7.18 (2H, d, J = 8.7 Hz), 7.19 (1H, brm), 7.30 (2H, d, J = 8.7 Hz), 7.54 (1H, d, J = 2.0 Hz), 7.74 (1H, d, J = 8.9 Hz), 12.09 (1H, s).
Moisture analysis according to Karl Fisher volumetric titration method: 4.27% (theoretical value of monohydrate: 3.99%)

[BRIEF DESCRIPTION OF THE DRAWINGS]

Fig. 1 is a diagram showing the effects of maintaining solubility of Compound A in the case of adding 5-{4-[(6-methoxy-1-methyl-1H-benzimidazol-2-yl)methoxy]benzyl}thiazolidine-2,4-dione hydrochloride (Compound A) and a formulation excipient (one excipient selected from the following excipients: hydroxypropyl cellulose, croscarmellose sodium, yellow ferric oxide, lactose and magnesium stearate) (Test Example 1).

Fig. 2 is a diagram showing the effects of maintaining solubility of Compound A in the case of adding 5-{4-[(6-methoxy-1-methyl-1H-benzimidazol-2-yl)methoxy]benzyl}thiazolidine-2,4-dione hydrochloride (Compound A) and a formulation excipient (one excipient selected from the following excipients: OPADRY II, polyvinyl alcohol and OPAGLOS 2) (Test Example 2).

## Claims

1. A pharmaceutical composition containing highly pure 5-{4-[(6-methoxy-l-methyl-1H-benzimidazol-2-yl)methoxy] benzyl}thiazolidine-2,4-dione, or a pharmacologically acceptable salt thereof, and one type or two or more types of excipients selected from pharmacologically acceptable cellulose, cellulose derivatives, polyvinyl alcohol derivatives and a polyvinyl alcohol derivative mixture.

2. The pharmaceutical composition according to Claim 1, wherein a decrease in solubility of the highly pure 5-{4-[(6-methoxy-1-methyl-1H-benzimidazol-2-yl)methoxy]benzyl}thiazolidine-2,4-dione, or pharmacologically acceptable salt thereof, is prevented.

3. The pharmaceutical composition according to Claim 1, wherein hydrate formation of the highly pure 5-{4-[(6-methoxy-1-methyl-1H-benzimidazol-2-yl)methoxy]benzyl}thiazolidine-2,4-dione or pharmacologically acceptable salt thereof, is prevented.

4. The pharmaceutical composition according to Claim 1, wherein superior in vivo absorbability of the 5-{4-[(6-methoxy-1-methyl-1H-benzimidazol-2-yl)methoxy]benzyl}thiazolidine-2,4-dione, or pharmacologically acceptable salt thereof, is maintained by preventing a decrease in the solubility of the highly pure 5-{4-[(6-methoxy-1-methyl-1H-benzimidazol-2-yl)methoxy]benzyl}thiazolidine-2,4-dione, or pharmacologically acceptable salt thereof.

5. The pharmaceutical composition according to Claim 1, wherein hydrate formation of the highly pure 5-{4-[(6-methoxy-1-methyl-1H-benzimidazol-2-yl)methoxy]benzyl}thiazolidine-2,4-dione, or pharmacologically acceptable salt thereof, is prevented in a water-containing solution.

6. The pharmaceutical composition according to Claim 1, wherein the highly pure 5-{4-[(6-methoxy-1-methyl-1H-benzimidazol-2-yl)methoxy]benzyl}thiazolidine-2,4-dione, or pharmacologically acceptable salt thereof, has superior solubility and stability of solubility over time in a water-containing solution.

7. The pharmaceutical composition according to Claim 1, wherein a decrease in solubility of the highly pure 5-{4-[(6-methoxy-1-methyl-1H-benzimidazol-2-yl)methoxy]benzyl}thiazolidine-2,4-dione, or pharmacologically acceptable salt thereof, is prevented in a water-containing solution.

8. The pharmaceutical composition according to Claim 1, wherein a decrease in solubility of the highly pure 5-{4-[(6-methoxy-1-methyl-1H-benzimidazol-2-yl)methoxy]benzyl}thiazolidine-2,4-dione, or pharmacologically acceptable salt thereof, is prevented in the digestive tract of a warm-blooded animal.

9. The pharmaceutical composition according to any one of Claims 1 to 8 that is a formulation for oral administration.

10. The pharmaceutical composition according to Claim 9, wherein the formulation for oral administration is a tablet.

11. The pharmaceutical composition according to any one of Claims 1 to 10, wherein the 5-{4-[(6-methoxy-1-methyl-1H-benzimidazol-2-yl)methoxy]benzyl}thiazolidine-2,4-dione, or pharmacologically acceptable salt thereof, is a purified composition in which the content of the 5-{4-[(6-methoxy-1-methyl-1H-benzimidazol-2-yl)methoxy]benzyl}thiazolidine-2,4-dione, or pharmacologically acceptable salt thereof, excluding water, is 98% by weight or more.

12. The pharmaceutical composition according to any one of Claims 1 to 10, wherein the 5-{4-[(6-methoxy-1-methyl-1H-benzimidazol-2-yl)methoxy]benzyl}thiazolidine-2,4-dione, or pharmacologically acceptable salt thereof, is a purified composition in which the content of the 5-{4-[(6-methoxy-1-methyl-1H-benzimidazol-2-yl)methoxy]benzyl}thiazolidine-2,4-dione, or pharmacologically acceptable salt thereof, excluding water, is 99% by weight or more.

13. The pharmaceutical composition according to any one of Claims 1 to 12, wherein the excipient is a pharmacologically acceptable cellulose or cellulose derivative.

14. The pharmaceutical composition according to Claim 13, wherein the pharmacologically acceptable cellulose or cellulose derivative is one or more types selected from methyl cellulose, hydroxymethyl cellulose, hydroxyethyl cellulose, hydroxyethyl methyl cellulose, hydroxypropyl cellulose, hydroxypropyl methyl cellulose 2208, hydroxypropyl methyl cellulose 2906, hydroxypropyl methyl cellulose 2910 and croscarmellose sodium.

**15.** The pharmaceutical composition according to any one of Claims 1 to 13, wherein the excipient is a pharmacologically acceptable polyvinyl alcohol derivative.

**16.** The pharmaceutical composition according to Claim 15, wherein the pharmacologically acceptable polyvinyl alcohol derivative is partially hydrolyzed polyvinyl alcohol.

**17.** The pharmaceutical composition according to any one of Claims 1 to 16, wherein the 5-{4-[(6-methoxy-1-methyl-1H-benzimidazol-2-yl)methoxy]benzyl}thiazolidine-2,4-dione, or pharmacologically acceptable salt thereof, is 5-{4-[(6-methoxy-1-methyl-1H-benzimidazol-2-yl)methoxy]benzyl}thiazolidine-2,4-dione hydrochloride.

**18.** The pharmaceutical composition according to any one of Claims 1 to 17 that is produced by a dry granulation process.

**19.** The pharmaceutical composition according to any one of Claims 1 to 17 that is produced by a wet granulation process.

**20.** The pharmaceutical composition according to any one of Claims 1 to 19 that is for the treatment of diabetes.

**21.** Use of 5-{4-[(6-methoxy-1-methyl-1H-benzimidazol-2-yl) methoxy]benzyl}thiazolidine-2,4-dione, or a pharmacologically acceptable salt thereof, and one type or two or more types of excipients selected from cellulose, cellulose derivatives, polyvinyl alcohol derivatives and polyvinyl alcohol derivative mixtures for manufacturing the pharmaceutical composition according to any one of Claims 1 to 20.

**22.** Use of 5-{4-[(6-methoxy-l-methyl-1H-benzimidazol-2-yl)methoxy]benzyl}thiazolidine-2,4-dione, or a pharmacologically acceptable salt thereof, and a pharmacologically acceptable cellulose or cellulose derivative for manufacturing the pharmaceutical composition according to any one of Claims 1 to 21.

**23.** Use of a purified composition, in which the content of 5-{4-[(6-methoxy-1-methyl-1H-benzimidazol-2-yl)methoxy] benzyl}thiazolidine-2,4-dione, or a pharmacologically acceptable salt thereof, excluding water, is 98% by weight or more, for manufacturing the pharmaceutical composition according to any one of Claims 1 to 21.

**24.** Use of the pharmaceutical composition according to any one of Claims 1 to 21 for treating diabetes.

**25.** Use of a pharmaceutical composition containing highly pure 5-{4-[(6-methoxy-1-methyl-1H-benzimidazol-2-yl)methoxy]benzyl}thiazolidine-2,4-dione, or a pharmacologically acceptable salt thereof, and a pharmacologically acceptable cellulose or cellulose derivative for treating diabetes.

**26.** A method for administering 5-{4-[(6-methoxy-1-methyl-1H-benzimidazol-2-yl)methoxy] benzyl}thiazolidine-2,4-dione, or a pharmacologically acceptable salt thereof, comprising: co-administering (i) a purified composition in which the content of 5-{4-[(6-methoxy-l-methyl-1H-benzimidazol-2-yl)methoxy]ben2.yl}thiazolidine-2,4-dione, or pharmacologically acceptable salt thereof, excluding water, is 98% by weight or more, and (ii) one type or two or more types selected from a polyvinyl alcohol derivative and a polyvinyl alcohol derivative mixture to a patient requiring the 5-{4-[(6-methoxy-1-methyl-1H-benzimidazol-2-yl)methoxy]benzyl}thiazolidine-2,4-dione, or a pharmacologically acceptable salt thereof.

**27.** The method according to Claim 26 for improving in vivo absorbability by maintaining the solubility of highly pure 5-{4-[(6-methoxy-1-methyl-1H-benzimidazol-2-yl)methoxy]benzyl}thiazolidine-2,4-dione, or a pharmacologically acceptable salt thereof.

**28.** A method for treating a human to treat or prevent diabetes, the method comprising: treating the human with a pharmaceutical composition containing (i) highly pure 5-{4-[(6-methoxy-1-methyl-1H-benzimidazol-2-yl)methoxy] benzyl}thiazolidine-2,4-dione, or a pharmacologically acceptable salt thereof, and (ii) one type or two or more types of excipients selected from cellulose, cellulose derivatives, polyvinyl alcohol derivatives and polyvinyl alcohol derivative mixtures.

**29.** The method according to Claim 28 comprising treating with a pharmaceutical composition containing (i) highly pure S-{4-[(6-methoxy-1-methyl-1H-benzimidazol-2-yl)methoxy]benzyl}thiazolidine-2,4-dione, or a pharmacologically acceptable salt thereof, and (ii) a pharmacologically acceptable cellulose or derivative thereof.

[Fig. 1]

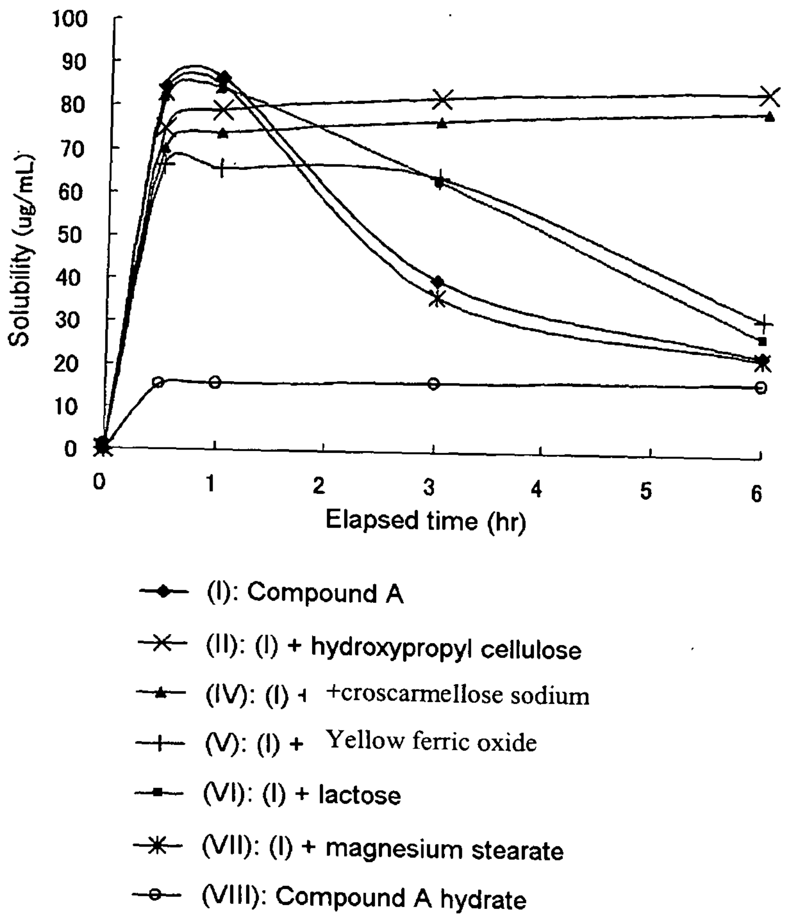
100
90
80
70
60
50
40
30
20
10
0
Solubility (ug/mL)
0
1
2
3
4
5
6
Elapsed time (hr)
(I): Compound A
(II): (I) + hydroxypropyl cellulose
(IV): (I) + +croscarmellose sodium
(V): (I) + Yellow ferric oxide
(VI): (I) + lactose
(VII): (I) + magnesium stearate
(VIII): Compound A hydrate

[Fig. 2]

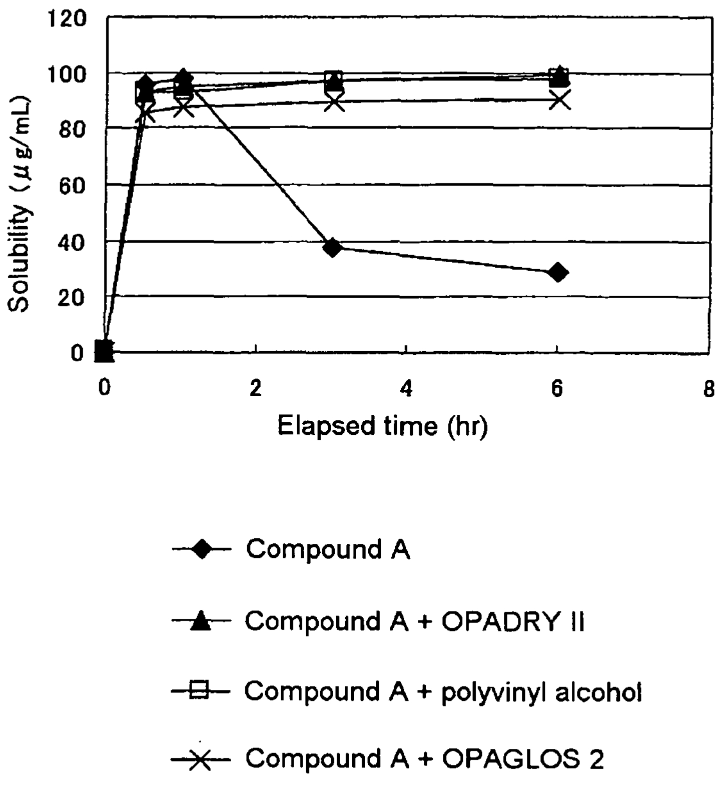
120
100
80
60
40
20
0
Solubility (μg/mL)
0
2
4
6
8
Elapsed time (hr)
Compound A
Compound A + OPADRY II
Compound A + polyvinyl alcohol
Compound A + OPAGLOS 2

**INTERNATIONAL SEARCH REPORT**

International application No.
PCT/JP2006/313548

A. CLASSIFICATION OF SUBJECT MATTER
*A61K31/427*(2006.01)i, *A61K9/20*(2006.01)i, *A61K47/32*(2006.01)i, *A61K47/38*(2006.01)i, *A61P3/10*(2006.01)i, *C07D417/12*(2006.01)i

According to International Patent Classification (IPC) or to both national classification and IPC

B. FIELDS SEARCHED

Minimum documentation searched (classification system followed by classification symbols)
A61K31/427, A61K9/20, A61K47/32, A61K47/38, A61P3/10, C07D417/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2002-220336 A (SANKYO CO., LTD.),<br>09 August, 2002 (09.08.02),<br>Claims; examples; pharmaceutical preparation example<br>(Family: none) | 1-13,17-23<br>1-23 |
| X<br>Y | WO 2000/071540 A1 (SANKYO CO., LTD.),<br>30 November, 2000 (30.11.00),<br>Claims; examples; pharmaceutical preparation example<br>& JP 2001-39976 A & CA 2375017 A1<br>& EP 1180519 A1 & US 2002/111373 A1 | 1-13,17-23<br>1-23 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 August, 2006 (07.08.06) | 15 August, 2006 (15.08.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/313548

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2004/000284 A1 (Transform Pharmaceuticals, Inc.), 31 December, 2003 (31.12.03), Full text & EP 1515703 A1 & JP 2006-500377 A & CN 1668283 A | 1-23 |
| Y | JP 7-291854 A (Tanabe Seiyaku Co., Ltd.), 07 November, 1995 (07.11.95), Claims (Family: none) | 1-23 |
| Y | JP 60-190723 A (Yamanouchi Pharmaceutical Co., Ltd.), 28 September, 1985 (28.09.85), Claims (Family: none) | 1-23 |

Form PCT/ISA/210 (continuation of second sheet) (April 2005)

**INTERNATIONAL SEARCH REPORT**

International application No.
PCT/JP2006/313548

**Box No. II Observations where certain claims were found unsearchable (Continuation of item 2 of first sheet)**

This international search report has not been established in respect of certain claims under Article 17(2)(a) for the following reasons:

1. [X] Claims Nos.: 24-29
because they relate to subject matter not required to be searched by this Authority, namely:
Claims 24 to 29 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0071540 A
- JP 2006301058 W
- JP 2001072671 A

### Non-patent literature cited in the description


- **LEON LACHMAN et al.** The Theory and Practice of Industrial Pharmacy. LEA & FEBIGER, 1986
- **HERBERT A. LIEBERMAN et al.** Pharmaceutical Dosage Forms: Tablets. MARCEL DEKKER INC, 1989, vol. 1